(19) Europäisches Patentamt
European Patent Office
Office européen des brevets

(11) Veröffentlichungsnummer : **0 028 392**
**B1**

(12) # EUROPÄISCHE PATENTSCHRIFT

(45) Veröffentlichungstag der Patentschrift : **16.02.83**

(51) Int. Cl.³ : **C 07 D265/36**

(21) Anmeldenummer : **80106628.3**

(22) Anmeldetag : **29.10.80**

(54) **Verfahren zur Herstellung von 2,3-Dioxo-1,4-benzoxazinderivaten.**

(30) Priorität : **06.11.79 DE 2944696**

(43) Veröffentlichungstag der Anmeldung : **13.05.81 Patentblatt 81/19**

(45) Bekanntmachung des Hinweises auf die Patenterteilung : **16.02.83 Patentblatt 83/07**

(84) Benannte Vertragsstaaten : **AT BE CH DE FR GB IT LI NL SE**

(56) Entgegenhaltungen :
**ANGEWANDTE CHEMIE, Band 92, Nr. 3, März 1980, Verlag Chemie GmbH, Weinheim, DE, G. REISSENWEBER et al. : « Oxidation von Isatinen zu Isatosäureanhydriden und 2,3-Dioxo-1,4-benzoxazinen », Seiten 196-197**

(73) Patentinhaber : **BASF Aktiengesellschaft**
**Carl-Bosch-Strasse 38**
**D-6700 Ludwigshafen (DE)**

(72) Erfinder : **Reissenweber, Gernot, Dr.**
**Pfarrer-Friedrich-Strasse 41**
**D-6700 Ludwigshafen (DE)**
Erfinder : **Mangold, Dietrich, Dr.**
**Hermann-Walker-Strasse 49**
**D-6903 Neckargemuend (DE)**

**EP 0 028 392 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 2,3-Dioxo-1,4-benzoxazinderivaten

Die vorliegende Erfindung betrifft ein Verfahren zur Herstellung von 2,3-Dioxo-1,4-benzoxazinderivaten durch Oxidation von Isatinen mit Peroxodisulfat in schwefelsaurem Medium.

Es ist bekannt, daß man durch Umsetzung von o-Aminophenolen mit Oxalylchlorid 2,3-Dioxo-1,4-benzoxazine erhält (Ann. Chem. *753*, 70-87 (1970)). Ebenso erhält man 2,3-Dioxo-1,4-benzoxazine, wenn man o-Nitrophenole mit Oxalesterchlorid umsetzt und anschließend die Nitrogruppe zur Aminogruppe reduziert, wobei unter Alkoholabspaltung Ringschluß zu den 2,3-Dioxo-1,4-benzoxazinderivaten erfolgt (DE-AS 1 161 080). Von Nachteil bei diesen Verfahren ist, daß o-Aminophenole und o-Nitrophenole, vor allem mit zusätzlichen Substituenten am Aromaten, meist nur unter großem Aufwand herstellbar sind und daß Oxalylchlorid und Oxalesterchlorid technisch nicht leicht zugänglich sind.

Es wurde nun gefunden, daß man 2,3-Dioxo-1,4-benzoxazinderivate der Formel I

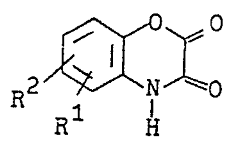

(I),

in der $R^1$ und $R^2$ gleich oder verschieden sind und Wasserstoff, Alkyl, Halogen, Halogenalkyl, Alkoxy, Halogenalkoxy oder Nitro bedeuten, in vorteilhafter Weise durch Oxidation von Isatinen der Formel II

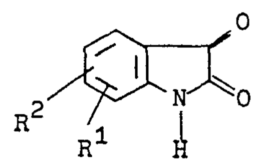

(II),

in der $R^1$ und $R^2$ die obenangegebenen Bedeutungen haben, mit Peroxodisulfat, gelöst oder suspendiert in 30- bis 98 %iger Schwefelsäure, erhält.

Im Vergleich zu den bekannten Verfahren liefert das erfindungsgemäße Verfahren, ausgehend von leicht zugänglichen Isatinen, in einfacher Weise und auf einem sehr wirtschaftlichen Weg die gewünschten Verbindungen in hoher Ausbeute und Reinheit.

Nach dem erfindungsgemäßen Verfahren sind außer 2,3-Dioxo-1,4-benzoxazin auch am Benzolkern substituierte Derivate herstellbar. Als Substituenten $R^1$ und $R^2$ kommen dabei unverzweigte oder verzweigte Alkyl-, Alkoxy-, Halogenalkyl- oder Halogenalkoxygruppen, insbesondere mit 1 bis 4 Kohlenstoffatomen, beispielsweise Methyl, Äthyl, Propyl- oder Butylreste, Methoxy, Äthoxy, i-Propoxy, n-Butoxy, sec.-Butoxy, i-Butoxy, Trifluormethyl, Difluormethyl, 2-Fluoräthyl, Trifluormethoxy, Tetrafluoräthoxy, oder Halogen, wie Chlor, Brom, Fluor, oder Nitro in Betracht.

Die Benzoxazinderivate der Formel I sind bekannte Pflanzenschutzmittel. So besitzen z. B. am Benzolkern chlorsubstituierte 2,3-Dioxo-1,4-benzoxazine ausgezeichnete fungizide bzw. fungistatische Eigenschaften (DE-AS 1 161 080).

Die Oxidation wird so durchgeführt, daß man 1 bis 2 Mol, vorzugsweise 1 bis 1,5 Mol, Peroxodisulfat, gelöst oder suspendiert in 750 bis 3 000 ml, vorzugsweise in 1 000 bis 2 000 ml, 30-bis 98 %iger Schwefelsäure löst und in diese Lösung bei – 20 °C bis + 30 °C, vorzugsweise bei 0 °C bis + 10 °C, langsam 1 Mol Isatin einträgt. Nach beendeter Zugabe läßt man das Reaktionsgemisch bei Verwendung von 80 bis 98 %iger Schwefelsäure noch 10 bis 30 Minuten, bei Verwendung von weniger konzentrierter Schwefelsäure 10,5 bis 24 Stunden nachrühren und gießt anschließend auf Eis. Das dabei kristallin anfallende Endprodukt kann nach den üblichen Methoden, z. B. durch Filtrieren oder Zentrifugieren, ohne Schwierigkeiten isoliert werden.

Geeignete Oxidationsmittel sind Salze der Peroxodischwefelsäure, wie Alkalimetallperoxodisulfate, beispielsweise Natrium- und Kaliumperoxodisulfat, oder Ammoniumperoxodisulfat sowie Peroxodischwefelsäure selbst.

Die als Ausgangsstoffe verwendeten kernsubstituierten Isatinderivate sind bekannt bzw. können in Anlehnung an bekannte Verfahren ohne Schwierigkeiten hergestellt werden (Houben-Weyl, Methoden der organ. Chemie, Bd. VII/4, S. 5-25, Georg Thieme-Verlag, Stuttgart, 1968).

Die folgenden Beispiele erläutern die Durchführung des erfindungsgemäßen Verfahrens. Die Mengenangaben sind Gewichtsteile.

## Beispiel 1

In eine Lösung aus 190 Teilen Kaliumperoxodisulfat in 1 300 Teilen 90 %iger Schwefelsäure trägt man

bei einer Temperatur zwischen 0 °C und 10 °C langsam 103 Teile Isatin ein. Man läßt 10 bis 20 Minuten nachrühren und gießt auf Eis. Nach dem Absaugen und Trocknen erhält man 110 Teile 2,3-Dioxo-1,4-benzoxazin vom Schmelzpunkt 285 °C (Zers.).

Beispiel 2

In eine Suspension aus 29 Teilen Kaliumperoxodisulfat und 280 Teilen 30 %iger Schwefelsäure trägt man bei Raumtemperatur 14,7 Teile Isatin ein. Man läßt ohne Kühlung 24 Stunden nachrühren und gießt anschließend das Reaktionsgemisch in Wasser. Nach dem Absaugen und Trocknen erhält man 11 Teile 2, 3-Dioxo-1,4-benzoxazin vom Schmelzpunkt 279-283 °C (Zers.).

Beispiel 3

In eine Lösung aus 56 Teilen Kaliumperoxodisulfat in 450 Teilen 92 %iger Schwefelsäure trägt man bei einer Temperatur zwischen 0 °C und 10 °C 32,2 Teile 7-Methylisatin ein. Man läßt wenige Minuten nachrühren und gießt anschließend auf Eis. Nach dem Absaugen und Trocknen erhält man 31,4 Teile 5-Methyl-2,3-dioxo-1,4-benzoxazin vom Schmelzpunkt 247-249 °C.

Beispiel 4

In eine Lösung aus 60 Teilen Kaliumperoxodisulfat in 400 Teilen 98 %iger Schwefelsäure trägt man bei einer Temperatur zwischen 5 °C und 10 °C 35 Teile 6,7-Dimethylisatin ein. Man läßt 10 bis 20 Minuten nachrühren und gießt anschließend auf Eis. Nach dem Absaugen und Trocknen erhält man 34 Teile 5,6-Dimethyl-2,3-dioxo-1,4-benzoxazin vom Schmelzpunkt 278-284 °C (Zers.).

Entsprechend können folgende 2,3-Dioxo-1,4-benzoxazine hergestellt werden :

7-Brom-2,3-dioxo-1,4-benzoxazin vom Schmp. 318 °C (Zers.) ; Ausbeute 95 % d. Th.

5,7-Dichlor-2,3-dioxo-1,4-benzoxazin vom Schmp. 257 °C ;
Ausbeute : 95 % d. Th.

7-Nitro-5-methyl-2,3-dioxo-1,4-benzoxazin vom Schmp.
275-283 °C ; Ausbeute : 85 % d. Th.

5-Trifluormethyl-2,3-dioxo-1,4-benzoxazin vom Schmp.
207-210 °C ; Ausbeute : 82 % d. Th.

**Ansprüche**

1. Verfahren zur Herstellung von 2,3-Dioxo-1,4-benzoxazinderivaten der Formel I

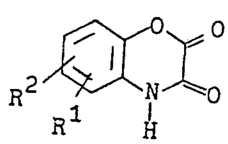

(I),

in der $R^1$ und $R^2$ gleich oder verschieden sind und Wasserstoff, Alkyl, Halogen, Halogenalkyl, Alkoxy, Halogenalkoxy oder Nitro bedeuten, dadurch gekennzeichnet, daß man Isatine der Formel II

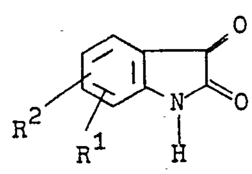

(II),

in der $R^1$ und $R^2$ die obenangegebenen Bedeutungen haben, mit der ein- bis zweifachen molaren Menge Peroxodisulfat, gelöst oder suspendiert in 750 bis 3 000 ml 30- bis 98 %iger Schwefelsäure, bezogen auf 1 bis 2 Mol Peroxodisulfat, bei − 20 bis + 30 °C oxidiert.

2. Verfahren zur Herstellung von 2,3-Dioxo-1,4-benzoxazinderivaten der Formel I nach Anspruch 1, dadurch gekennzeichnet, daß man die Oxidation mit einem Alkalimetallperoxodisulfat durchführt.

**Claims**

1. A process for the preparation of a 2,3-dioxo-1,4-benzoxazine derivative of the formula I

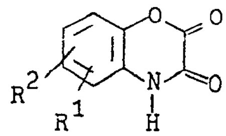

(I)

where $R^1$ and $R^2$ are identical or different and each is hydrogen, alkyl, halogen, haloalkyl, alkoxy, haloalkoxy or nitro, wherein an isatin of the formula II

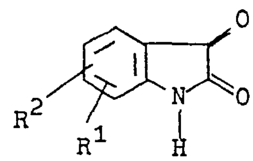

(II),

where $R^1$ and $R^2$ have the above meanings, is oxidized with one to two times the molar amount of peroxydisulfate, dissolved or suspended in 750-3 000 ml of 30-98 % strength sulfuric acid, with reference to 1 to 2 moles of peroxydisulfate, at − 20 to + 30 °C.

2. A process for the preparation of a 2,3-dioxo-1,4-benzoxazine derivative of the formula I as claimed in claim 1, wherein the oxidation is carried out with an alkali metal peroxydisulfate.

**Revendications**

1. Procédé de préparation de dérivés de 2,3-dioxo-1,4-benzoxazine de la formule I

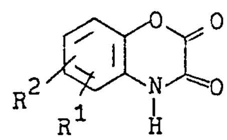

(I),

dans laquelle $R^1$ et $R^2$, qui peuvent être identiques ou différents, désignent chacun un atome d'hydrogène ou d'halogène, un radical alcoyle, alcoxy ou halo-alcoxy ou un groupe nitro, caractérisé en ce que l'on oxyde des isatines de la formule II

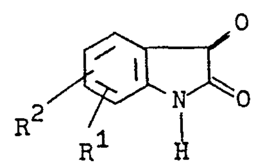

(II),

dans laquelle $R^1$ et $R^2$ possèdent les significations définies ci-dessus, entre − 20 et + 30 °C à l'aide de 1 à 2 moles d'un peroxodisulfate, dissous ou mis en suspension dans 750 à 3 000 ml d'un acide sulfurique d'une concentration de 30 à 98 % (pour 1 à 2 moles de peroxodisulfate).

2. Procédé de préparation de dérivés de 2,3-dioxo-1,4-benzoxazine de la formule I selon la revendication 1, caractérisé en ce que l'oxydation est réalisée à l'aide d'un peroxodisulfate d'un métal alcalin.